# EUROPEAN PATENT SPECIFICATION

(11) **EP 3 749 214 B1**
(45) Date of publication and mention of the grant of the patent: **22.09.2021**
(21) Application number: 19706250.8
(22) Date of filing: 07.02.2019
(51) Int. Cl.: A61B 8/12, A61B 8/00, A61B 1/005

(54) **DEVICES, SYSTEMS, AND METHODS FOR TRANSESOPHAGEAL ECHOCARDIOGRAPHY**
VORRICHTUNGEN, SYSTEME UND VERFAHREN FÜR TRANSÖSOPHAGEALE ECHOKARDIOGRAFIE
DISPOSITIFS, SYSTÈMES ET PROCÉDÉS D'ÉCHOCARDIOGRAPHIE TRANSOESOPHAGIENNE

(30) Priority: 08.02.2018 US 201862627968 P
(43) Date of publication of application: 16.12.2020
(73) Proprietor: Koninklijke Philips N.V., 5656 AG Eindhoven (NL)
(72) Inventor: CASWELL, John, Bench, 5656 AE Eindhoven (NL); CUSCUNA, Dino, Francesco, 5656 AE Eindhoven (NL); KANAKASABHAPATHI, Sujith, 5656 AE Eindhoven (NL); LEDOUX, Nikolas, Keith, 5656 AE Eindhoven (NL); PESZYNSKI, Michael, 5656 AE Eindhoven (NL); POLAND, Mckee, Dunn, 5656 AE Eindhoven (NL); WIGHT, Michael, J., 5656 AE Eindhoven (NL)
(74) Representative: Philips Intellectual Property & Standards
(86) International application number: PCT/EP2019/053065
(87) International publication number: WO 2019/154943

(56) References cited:
- WO-A2-2010/020939
- US-A1- 2009 118 618
- US-A1- 2017 007 202

## Description

### TECHNICAL FIELD

The present disclosure relates generally to the inspection and imaging of the organs of a patient using transesophageal echocardiography (TEE). For example, some embodiments of the present disclosure are suited for modularly adapting TEE probes to be used with one or more modular gastroscopes. Other embodiments of the present disclosure are suited for wireless TEE procedures.

### BACKGROUND

Observing the condition and function of a patient's heart can be a difficult and dangerous procedure. Echocardiography can mitigate risk of injury to the patient by using ultrasonic imaging techniques. In an echocardiogram, a physician uses an ultrasonic probe comprising one or more ultrasonic transducers to obtain images of various angles of the patient's heart. The ultrasonic transducers emit ultrasonic energy in the form of ultrasonic waves to create an image of the heart. Ultrasonic waves are partially reflected by discontinuities arising from tissue structures, red blood cells, and other features of interest. Echoes, or the reflected ultrasonic waves, are received by the ultrasonic transducer and transmitted to a signal processor. The signal processor processes the received ultrasound echoes to produce an image of the heart near the location where the ultrasonic transducer is placed.

One common echocardiography procedure is a transthoracic echocardiogram (TTE), which involves placing an ultrasonic probe on the chest or abdomen of a patient to obtain images of various angles of the patient's heart. Although TTE is a relatively non-invasive procedure, the ultrasonic waves must travel through several layers of tissue and bone to reach the heart, and the echoes must travel back through the same tissue and bone to reach the ultrasonic transducer. These thick layers of tissue and bone can weaken the strength of the echoes and degrade the quality of the image obtained.

Transesophageal echocardiography (TEE) involves obtaining an ultrasonic image of the heart using a TEE probe positioned within the patient's esophagus. TEE probes also use ultrasonic waves to obtain an image of a patient's heart. Transesophageal echocardiography can be advantageous because the heart is located near the esophagus, which can result in higher quality images. To obtain an image using a TEE probe, a physician inserts a gastroscope including an ultrasonic transducer into the patient's esophagus and guides the ultrasonic transducer to an area near the heart. The physician manipulates a distal portion of the gastroscope within the patient's esophagus to guide the ultrasonic transducer into place, and to maintain contact with the esophagus wall to facilitate the transmission and reception of the ultrasonic waves.

Conventional TEE probes include a handle and a gastroscope having an ultrasound transducer and an elongated body coupled to the handle. The handle may also be coupled to an interface cable leading to a console. In some conventional embodiments, the handle, gastroscope, and console interface cable comprise an integral unit in which the separate components are permanently fixed to one another. In other words, in conventional embodiments, the gastroscope is not configured to be repeatedly separated from and recoupled to a handle by a physician or other user.

The size and shape of the gastroscope portion of the TEE probe that the physician uses to obtain ultrasonic images may depend on the physiology of the patient and the particular type of image desired by the physician. In other words, when a physician desires to obtain an ultrasonic image of a patient's heart, she must select a gastroscope that matches the size and configuration of the patient's physiology. Because conventional TEE probes include permanently fixed gastroscopes and other components, the physician must have a separate TEE probe, including a handle and a console interface cable, for each gastroscope configuration she may use in her practice. This is true even though the handle and console interface cable do not vary between gastroscopes.

Cleaning conventional TEE probes can be difficult and damaging to TEE probe components. For example, the gastroscope, which contacts the patient's physiology, may require more rigorous cleaning while the handle and console interface cable may require less rigorous cleaning. Procedures suitable to clean one component of the TEE probe, may damage other parts of the TEE probe. The complexity of cleaning a TEE probe with components of differing resilience can lead to time-consuming and cumbersome cleaning procedures and damage to TEE probe components. When conventional TEE probe components are damaged, the entire TEE probe, including the handle and console interface cable, must be sent away for repair, or more expensively, for replacement.

Furthermore, in a conventional transesophageal echocardiogram, the physician uses x-ray imaging on the patient's esophagus to guide the TEE probe to the desired location, subjecting the physician to radiation. Because the controls for conventional TEE probes are placed on the handle, the physician must maintain a constant and steady grip on the handle of the TEE probe for long periods of time, and often at awkward angles.

US 2009/0118618 discloses an ultrasound probe having a handle, a flexible shaft and an ultrasound transducer. The flexible shaft and transducer are detachable from the handle.

WO 2010/020939 discloses an ultrasound transducer with a wireless interface.

US 2017/007202 discloses a TEE probe with haptic feedback from the distal tip.

### SUMMARY

The invention is defined by the claims.

Embodiments of the present disclosure provide an improved transesophageal echocardiography (TEE) probe for generating images of an organ. For example, the TEE probe can include components, such as a gastroscope and a handle, that are removably coupled to another to allow for detachment and substitution of one or more components. The systems, devices and methods described herein advantageously allow for a TEE probe to be disassembled and reassembled by a physician or user, using the same or different components. This advantageously improves the medical workflow for the patient and the physician, and mitigates damage to the TEE probe during cleaning procedures.

The present disclosure also describes TEE probes that can be controlled remotely, or wirelessly. Embodiments of the present disclosure also provide a TEE probe including a wireless module configured to receive and transmit electrical signals. For example, the wireless module can be configured to receive a command signal from a remote user, permitting the remote user to remotely or wirelessly control various aspects of the TEE procedure. The wireless module may also be configured to transmit ultrasonic imaging data to a console at or near the remote user to allow the remote user to observe an ultrasonic image. This can reduce the physician's exposure to radiation in an operating room, and can reduce the difficulty of the TEE procedure. Additionally, a TEE probe having a wireless module can include removable components as described above, thus enjoying both the benefits of the wireless module and the removable or modular components.

According to an exemplary embodiment, a transesophageal echocardiography (TEE) probe is provided. The TEE probe includes a handle comprising a proximal portion, a distal portion, and a handle-mating interface disposed at the distal portion. The TEE probe can further comprise a gastroscope coupled to the handle and configured to be positioned within an esophagus of a patient. The gastroscope can comprise a proximal portion, a distal portion, an ultrasonic transducer disposed at the distal portion of the gastroscope and configured to obtain ultrasonic imaging data, and a gastroscope-mating interface disposed at the proximal portion of the gastroscope. The handle and the gastroscope can be removably coupled via the handle-mating interface and the gastroscope-mating interface, such that when the handle-mating interface and the gastroscope-mating interface are coupled, the handle-mating interface is configured to transmit an electrical signal to the gastroscope via the gastroscope-mating interface to control movement of the distal portion of the gastroscope.

In some embodiments, the ultrasonic transducer comprises an ultrasonic transducer array, and the gastroscope comprises a microbeamformer in communication with the ultrasonic transducer array. The microbeamformer can be disposed at the distal portion of the gastroscope. In some embodiments, the handle comprises a beamformer in communication with the ultrasonic transducer array. In other embodiments, the gastroscope comprises a microbeamformer in communication with the ultrasonic transducer array and the beamformer. The handle-mating interface and the gastroscope-mating interface can comprise pogo pin interfaces, in some instances. At least one of the handle or the gastroscope can comprise a latch to secure the handle-mating interface and the gastroscope-mating interface.

In some embodiments, the gastroscope comprises an actuator and a pull cable coupled to the actuator and the distal portion of the gastroscope. The actuator can be configured to control movement of the distal portion of the gastroscope. The handle may comprise a controller in communication with the actuator and the ultrasonic transducer. The actuator can include a motor. The gastroscope may include a force sensor disposed at the distal portion of the gastroscope, and can be configured to detect a force applied to the distal portion of the gastroscope. In some instances, the TEE probe further includes a force sensor controller in communication with the force sensor and the actuator. The force sensor controller can be configured to control actuation of the pull cable by the actuator based on a force detected by the force sensor. The force sensor can comprise a flexible substrate positioned around the distal portion of the gastroscope. In other embodiments, the handle comprises a console-mating interface at the proximal portion of the handle. The console-mating interface may comprise at least one of a USB interface or a pogo pin interface.

In other embodiments, a TEE probe includes a gastroscope configured to be coupled to a handle and positioned within an esophagus of a patient. The gastroscope can comprise a distal portion, a proximal portion, an ultrasonic transducer disposed at the distal portion of the gastroscope and configured to obtain ultrasonic imaging data, a motor, and a pull cable. The pull cable is coupled to the motor and the distal portion of the gastroscope, in some instances. The motor may be configured to receive an electrical signal from the handle to control movement of the distal portion of the gastroscope using the pull cable. In some embodiments the gastroscope comprises a force sensor configured to detect a force applied to the distal portion of the gastroscope. The TEE probe can also comprise a force sensor controller in communication with the force sensor and the motor. The force sensor controller may be configured to control actuation of the pull cable by the motor based on a force detected by the force sensor. In other embodiments, the TEE probe comprises a motor sensor in communication with the motor and configured to detect a position of the motor. In still other embodiments, the ultrasonic transducer comprises an ultrasonic transducer array, and the gastroscope comprises a microbeamformer that is in communication with the ultrasonic transducer array and disposed at the distal portion of the gastroscope.

According to a further exemplary embodiment, a TEE probe is provided. The TEE probe includes a handle, a gastroscope coupled to the handle, and a wireless module coupled to the handle and in communication with the gastroscope. The gastroscope can be configured to be positioned within an esophagus of a patient, and can include an ultrasonic transducer disposed at a distal portion of the gastroscope. The gastroscope can be configured to obtain ultrasonic imaging data. The wireless module can include a wireless communication element, and a microcontroller in communication with the wireless communication element. The wireless module can be configured to receive a command signal from a console spaced from the TEE probe and to transmit the ultrasonic imaging data to the console. The microcontroller, in response to the received command signal, may be configured to transmit an electrical signal to the gastroscope to control the distal portion of the gastroscope.

In some embodiments, the TEE probe further comprises a user interface coupled to the handle and configured to receive a manual input from a user to control the gastroscope. In other embodiments, the microcontroller is configured to transmit the electrical signal to control movement of the distal portion of the gastroscope, in response to the command signal. In yet other embodiments, the gastroscope comprises a pull cable coupled to the distal portion of the gastroscope, and an actuator coupled to the pull cable. The microcontroller can be configured to transmit the electrical signal to the actuator to actuate the pull cable to move the distal portion of the gastroscope. In some embodiments, the microcontroller is configured to transmit the electrical signal to control the ultrasonic transducer in response to the command signal. In other embodiments, the ultrasonic transducer comprises an ultrasonic transducer array, and the handle comprises a beamformer in communication with the ultrasonic transducer array. The handle can also comprise a signal processor in communication with the beamformer. In some instances, the gastroscope comprises a microbeamformer in communication with the ultrasonic transducer array and the beamformer. The TEE probe can further comprise a battery configured to power the wireless module and the gastroscope, in some instances.

In some embodiments, the TEE probe comprises a handle-mating interface disposed at a distal portion of the handle, and a gastroscope-mating interface disposed at a proximal portion of the gastroscope. The handle and the gastroscope can be removably coupled via the handle-mating interface and the gastroscope-mating interface. The handle-mating interface can be configured to transmit the electrical signal to the gastroscope via the gastroscope-mating interface to control the distal portion of the gastroscope. In some embodiments, the wireless module is disposed within the handle. In other embodiments, the wireless module comprises a housing coupled to the handle, such that when the wireless module is coupled to the handle, the wireless module transmits the electrical signal to the gastroscope. In some embodiments, the housing removable couples to the handle by at least one of a USB interface or a pogo pin interface.

According to an exemplary embodiment, a method for wirelessly controlling a transesophageal echocardiography (TEE) probe is provided. The method can include: wirelessly receiving, by a wireless communication element coupled to a handle of the TEE probe, a command signal transmitted by a console spaced from the TEE probe while a gastroscope of the TEE probe is positioned within an esophagus of a patient; transmitting, by the wireless communication element, the received command signal to a controller coupled to the handle; and applying, by the controller in response to the received command signal, a voltage to an actuator coupled to the gastroscope to control movement of a distal portion of the gastroscope within the esophagus.

In some embodiments, the method can further comprise receiving, at the controller coupled to the handle, a feedback signal from the actuator, and modifying, by the actuator and based on the feedback signal, the movement of the distal portion of the gastroscope. In some aspects, the step of receiving the feedback signal from the actuator comprises receiving a force detection signal, and the step of modifying movement of the distal portion of the gastroscope comprises halting, by the actuator, the movement of the distal portion of the gastroscope. In some embodiments, the method further comprises wirelessly transmitting, by the wireless communication element to the console, the feedback signal, and activating a force detection indicator in response to the received feedback signal. The feedback signal may comprise a force detection signal. In some embodiments, the method further comprises: obtaining, by an ultrasonic transducer at the distal portion of the gastroscope, ultrasonic imaging data; and wirelessly transmitting, by the wireless communication element, the ultrasonic imaging data to the console. The method can further comprise receiving the ultrasonic imaging data obtained by the ultrasonic transducer at a beamformer, and transmitting the beamformed ultrasonic imaging data to the wireless communication element. In some embodiments, the method further comprises receiving ultrasonic imaging data obtained by the ultrasonic transducer at a microbeamformer and transmitting the microbeamformed ultrasonic imaging data to the microbeamformer.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a side elevation view of a prior art transesophageal echocardiography (TEE) probe, according to one embodiment.
Fig. 2 is a diagrammatic view of the prior art TEE probe of Fig. 1.
Fig. 3 is a perspective view of a modular ultrasonic imaging device and ultrasonic imaging system according to an embodiment of the present disclosure.
Fig. 4 is a diagrammatic view of a TEE probe system according to an embodiment of the present disclosure.
Fig. 5A is a perspective view of a handle of a TEE probe according to an embodiment of the present disclosure.
Fig. 5B is a partially transparent perspective view of the handle of Fig. 5A according to an embodiment of the present disclosure.
Fig. 6A is a perspective view of a proximal portion of a gastroscope, a gastroscope-mating interface, and a distal portion of a handle of a TEE probe according to an embodiment of the present disclosure, wherein the gastroscope and the handle are uncoupled.
Fig. 6B is a perspective view of a proximal portion of a gastroscope, a handle-mating interface, and a distal portion of a handle of a TEE probe according to an embodiment of the present disclosure, wherein the gastroscope and the handle are uncoupled.
Fig. 7 is a perspective view of the TEE probe of Figs. 6A and 6B, wherein the gastroscope and the handle are coupled.
Fig. 8 is a partially transparent perspective view of a proximal portion of a gastroscope and a handle according to an embodiment of the present disclosure.
Fig. 9A is a side elevation view of a lateral side of a distal portion of a TEE probe according to an embodiment of the present disclosure, illustrating anterior and posterior flexion of the distal portion.
Fig. 9B is a side elevation view of a front side of the distal portion of the TEE probe of Fig. 9A, illustrating left and right flexion of the distal portion.
Fig. 9C is a side elevation view of the front side of the distal portion of the TEE probe of Fig. 9A, illustrating counterclockwise, clockwise rotation, advanced and withdrawn positioning of the distal portion.
Fig. 9D is a perspective view of the distal portion of the TEE probe of Fig. 9A, illustrating increasing and decreasing multi-plane angles of an ultrasonic transducer.
Fig. 10 is a cross-sectional view of a distal portion of a handle and a proximal portion of a gastroscope of a TEE probe according to an embodiment of the present disclosure.
Fig. 11 is a cross-sectional view of a distal portion of a gastroscope of a TEE probe according to an embodiment of the present disclosure.
Fig. 12 is a schematic view of a TEE probe system according to an embodiment of the present disclosure.
Fig. 13 is a schematic view of a TEE probe system including a microbeamformer according to another embodiment of the present disclosure.
Fig. 14 is a perspective view of a proximal portion of a handle of a TEE probe having a console interface according to an embodiment of the present disclosure.
Fig. 15 is a partially transparent perspective view of a wireless module of a TEE probe according to an embodiment of the present disclosure.
Fig. 16 is a diagrammatic view of a TEE probe system comprising a wireless module, according to an embodiment of the present disclosure.
Fig. 17 is a diagrammatic view of a TEE probe system comprising a wireless module, according to another embodiment of the present disclosure.
Fig. 18 is a diagrammatic view of a method for remotely controlling a TEE procedure according to an embodiment of the present disclosure.
Fig. 19 is a diagrammatic view of a method for obtaining and wirelessly transmitting an ultrasonic image to a console.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

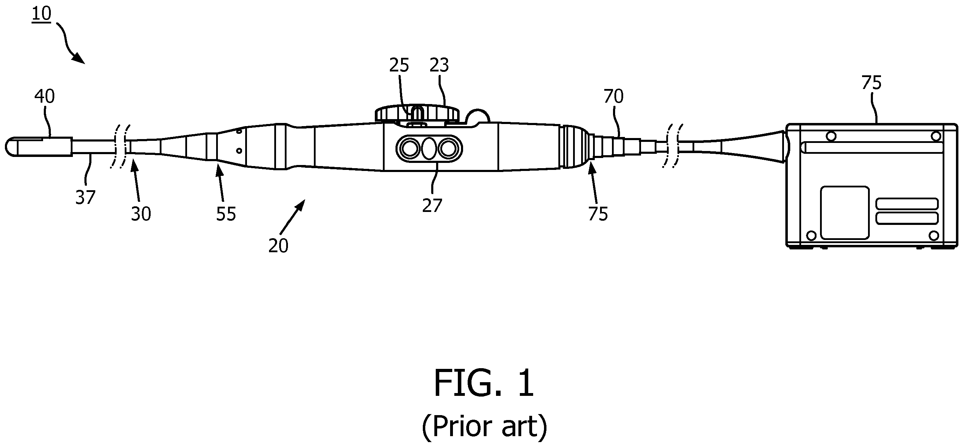

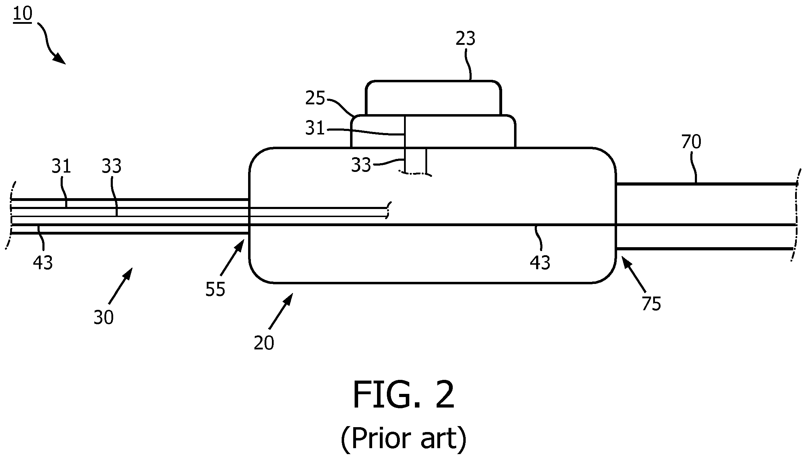

Figs. 1 and 2 depict a conventional TEE probe 10, including a gastroscope 30, a handle 20, and a console interface cable 70. In this embodiment, the gastroscope 30, handle 20, and console interface cable 70 are permanently connected. The handle 20 is permanently connected to the gastroscope at permanent connection 55, and to the console interface cable 70 at permanent connection 75. In other words, the gastroscope 30 and console interface cable 70 of the TEE probe 10 of Figs. 1 and 2 are not configured to separate or detach from the handle 20, and vice versa. In some embodiments, a conventional TEE probe 10 may comprise an integral unit wherein the gastroscope 30 and console interface cable 70 are permanently fixed to the handle 20 at the permanent connections 55, 75 by adhesives, screws, or other means of attachment which are not readily separated by a physician or user.

Permanent may be used to refer broadly to a mode of coupling or attachment that is not configured or designed for regular detachment and reattachment. Thus, although two coupled components can be physically separated, this disclosure may refer to the coupling or attachment as permanent when the components are not detached in the ordinary medical usage of the device. The terms coupled, coupling, coupled to, or connected, are used to refer broadly to any combination, attachment, or connection. This disclosure may refer to two components as coupled, even though they are permanently fixed to one another, or integrally formed as a single unit. Coupled, as used in this disclosure, contemplates direct and indirect modes of coupling or attachment, and modes of coupling or attachment that are removable. Two components may be referred to as coupled to one another even though they do not directly contact one another, or there are one or more conjoining components between the two components. Removable or separable may be used to refer to a mode of coupling or attachment that is configured to be separated and reattached in the normal course of use of a device or system, such as before, during, or after a medical procedure.

The gastroscope 30 includes an elongate body 37, a distal portion 39, and a proximal portion 32. The elongate body 37 may be flexible to be guided through the esophagus of the patient. The gastroscope 30 includes first and second pull cables 31, 33 that are mechanically connected to first and second movement controls 23, 25 coupled to the handle 20. Such a configuration may enable a physician to control movement of the distal portion of the gastroscope. Controlling movement of the distal portion 39 of the gastroscope 30 can aid the physician to guide the gastroscope 30 through the physiology of the patient to the desired imaging location. A rigid or fixed configuration of a gastroscope could injure the patient, and may be unsuitable for ultrasonic imaging, which demands accurate placement and sufficient contact with the patient's physiology to obtain ultrasonic images. Thus, pull cables 31, 33 and flexible components aid the physician in obtaining ultrasonic images of the patient's organs. Because the first and second pull cables 31, 33 are mechanically connected to the first and second movement controls 25, 27, a physician manipulating the first and second movement controls 23, 25 may receive tactile feedback from the distal portion 39 of the gastroscope 30 when the distal portion 39 presses against the walls of the esophagus.

Because conventional pull cables are manipulated by pull wires that extend from the gastroscope tip to movement controls disposed at the handle, gastroscopes cannot be separated or detached from the handle in the normal course of use. Thus, gastroscopes have typically been permanently fixed to a handle. But such configurations can lend themselves to disadvantages like those discussed above, such as unnecessary redundancy of TEE probe hardware, and costly or risky cleaning procedures.

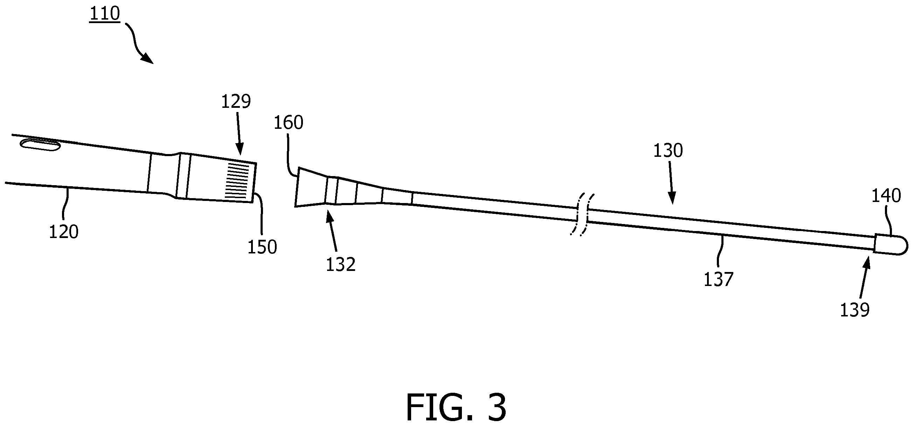

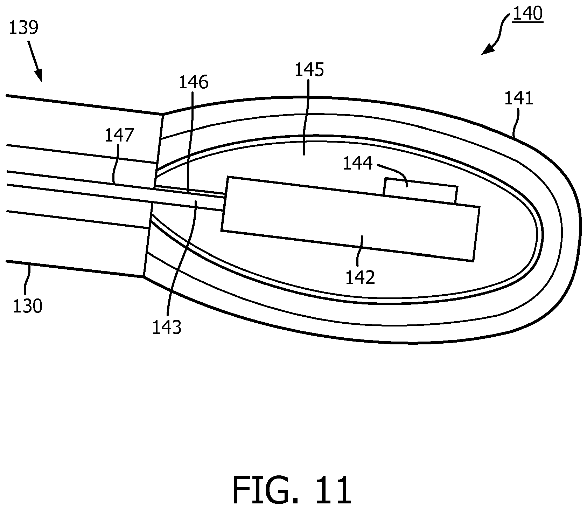

Fig. 3 depicts an ultrasound device 110 of an ultrasonic imaging system 100, according to one embodiment of the present disclosure. In the illustrated embodiment, the ultrasonic imaging system device comprises a TEE probe 110. In this embodiment, a gastroscope 130, having a proximal portion 132 and a distal portion 139, is shown detached from a handle 120. The gastroscope 130 includes a gastroscope tip 140 at the distal portion 139 of the gastroscope 130. The gastroscope 130 includes a gastroscope-mating interface 160 at the proximal portion 132 of the gastroscope 130, and the handle 120 includes a handle-mating interface 150 at a distal portion 129 of the handle 120. The gastroscope 130 and the handle 120 may be configured to removably couple to one another via the handle-mating interface 150 and the gastroscope-mating interface 160. In some embodiments, the gastroscope 130 and handle 120 removably couple to one another such that a user can control movement of the distal portion 139 of the gastroscope 130 by one or more user controls of the handle, and such that ultrasonic imaging data and/or control data can travel between an ultrasonic transducer of the gastroscope 130 across the gastroscope-mating interface 160 and handle-mating interface 150 to a console. In some embodiments, the gastroscope tip 140 includes an imaging element configured to obtain imaging data associated with anatomy within which the gastroscope 130 is positioned. For example, the imaging element can include one or more ultrasonic transducers (e.g., imaging element 142, Fig. 11) is configured to obtain ultrasonic imaging data. For example, the imaging element can be an ultrasonic transducer array including one or more ultrasonic transducer elements. For example, in some embodiments, an ultrasonic transducer array comprises between 2 and 1100 ultrasonic transducer elements. In some embodiments, an ultrasonic transducer array comprises 64, 128, 512, or 1024 ultrasonic transducer elements, or any other suitable number of ultrasonic transducer elements, both larger and smaller.

As used with respect to this and other embodiments, handle may refer to a structure configured to be gripped, manipulated, or manually controlled by a physician. As described in further detail below, a handle may also be configured to perform various and functions and operations, and may comprise electronic components. For example, manipulating the distal portion 139 of the gastroscope 130 may involve manipulating a dial or other user control of the handle 120 that can retract and/or release one or more pull cables 131, 133 coupled to the distal portion of the gastroscope. For example, retracting and releasing the one or more pull cables can control the posterior/anterior flexion or the right/left flexion of the distal portion of the gastroscope. Thus, although the handle 120 shown in Fig. 3 includes a body or housing configured to be gripped or handled by a physician, this disclosure uses handle to refer broadly to a part of a TEE probe system which may comprise various components, such as electronics.

Although the ultrasound device 110 discussed with respect to Fig. 3 and other figures of the present disclosure is a TEE probe, it is within the scope of this disclosure to provide various ultrasonic imaging devices and systems configured to obtain ultrasound images of various parts of a patient's anatomy. Accordingly, the ultrasound device 110 can be any type of imaging system suitable for use in various body lumens of a patient. In some embodiments, the ultrasound device 110 may include systems configured for forward looking intravascular ultrasound (IVUS) imaging, intravascular ultrasound (FL-IVUS) imaging, intravascular photoacoustic (IVPA) imaging, intracardiac echocardiography (ICE), and/or other suitable imaging modalities.

It is understood that the ultrasound device 110 can be configured to obtain any suitable intraluminal imaging data. In some embodiments, the device 110 can include an imaging component of any suitable imaging modality, such as optical imaging, optical coherence tomography (OCT), etc. In some embodiments, the device 110 can be configured to obtain any suitable intraluminal data using a pressure sensor, a flow sensor, a temperature sensor, an optical fiber, a reflector, a mirror, a prism, an ablation element, a radio frequency (RF) electrode, a conductor, and/or combinations thereof. Generally, the device 110 can include an electronic, mechanical, optical, and/or acoustic sensing element to obtain intraluminal data associated with a body lumen of a patient. The device 110 may be sized and shaped, structurally arranged, and/or otherwise configured for insertion into a lumen of the patient.

In some illustrated embodiments, the ultrasound device 110 is a TEE probe. In some embodiments, the ultrasound device is a catheter, a guide catheter, or a guide wire. The ultrasound device 110 can include a flexible elongate member 137. As used herein, elongate member or flexible elongate member includes at least any thin, long, flexible structure structurally arranged (e.g., sized and/or shaped) to be positioned within a lumen of the anatomy. For example, a distal portion 139 of the flexible elongate member 137 can be positioned within a lumen, while a proximal portion 132 of the flexible elongate member 137 can be positioned outside of the body of the patient. The flexible elongate member 137 can include a longitudinal axis. In some instances, the longitudinal axis can be a central longitudinal axis of the flexible elongate member 137. In some embodiments, the flexible elongate member 137 can include one or more polymer/plastic layers formed of various grades of nylon, Pebax, polymer composites, polyimides, and/or Teflon. In some embodiments, the flexible elongate member 137 can include one or more layers of braided metallic and/or polymer strands. The braided layer(s) can be tightly or loosely braided in any suitable configuration, including any suitable per in count (pic). In some embodiments, the flexible elongate member 137 can include one or more metallic and/or polymer coils. All or a portion of the flexible elongate member 137 may have any suitable geometric cross-sectional profile (e.g., circular, oval, rectangular, square, elliptical, etc.) or non-geometric cross-sectional profile. For example, the flexible elongate member 137 can have a generally cylindrical profile with a circular cross-sectional profile that defines an outer diameter of the flexible elongate member 137. For example, the outer diameter of the flexible elongate member 137 can be any suitable value for positioning within the anatomy 102, including between approximately 1 Fr and approximately 80 Fr, including values such as 3 Fr, 7 Fr, 8.2 Fr, 9 Fr, 25 Fr, 30 Fr, 34 Fr, 51 Fr, 60 Fr, and/or other suitable values both larger and smaller.

The ultrasound device 110 may or may not include one or more lumens extending along all or a portion of the length of the flexible elongate member 137. The lumen of the ultrasound device 110 can be structurally arranged (e.g., sized and/or shaped) to receive and/or guide one or more other diagnostic and/or therapeutic instruments. If the ultrasound device 110 includes lumen(s), the lumen(s) may be centered or offset with respect to the cross-sectional profile of the device 110. In some embodiments, the ultrasound device 110 may be used in conjunction with a guide wire. Generally, a guide wire is a thin, long, flexible structure that is structurally arranged (e.g., sized and/or shaped) to be disposed within the lumen of the anatomy. During a diagnostic and/or therapeutic procedure, a medical professional typically first inserts the guide wire into the lumen of the anatomy and moves the guide wire to a desired location within the anatomy, such as adjacent to an intravascular occlusion. The guide wire facilitates introduction and positioning of one or more other diagnostic and/or therapeutic instruments, including the ultrasound device 110, at the desired location within the anatomy. For example, the ultrasound device 110 moves through the lumen of the anatomy along the guide wire. In some embodiments, the lumen of the ultrasound device 110 can extend along the entire length of the flexible elongate member 137. In some embodiments, the ultrasound device 110 is not used with a guide wire, and the exit/entry port can be omitted from the ultrasound device 110.

The anatomy may represent any fluid-filled or surrounded structures, both natural and man-made. For example, the anatomy can be within the body of a patient. Fluid can flow through the lumen of the anatomy. In some instances, the ultrasound device 110 can be referenced as a transesophageal device. The anatomy can be the mouth, throat, esophagus, and/or stomach of a patient. In other instances, the ultrasound device 110 can be referenced as an intracardiac or intravascular device. In various embodiments, the anatomy is an artery or a vein of a patient's vascular system, including cardiac vasculature, peripheral vasculature, neural vasculature, renal vasculature, and/or any other suitable anatomy/lumen inside the body. The anatomy can be tortuous in some instances. For example, the device 110 may be used to examine any number of anatomical locations and tissue types, including without limitation, organs including the liver, heart, kidneys, gall bladder, pancreas, lungs, esophagus; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 110 may be used to examine man-made structures such as, but without limitation, heart valves, stents, shunts, filters and other devices.

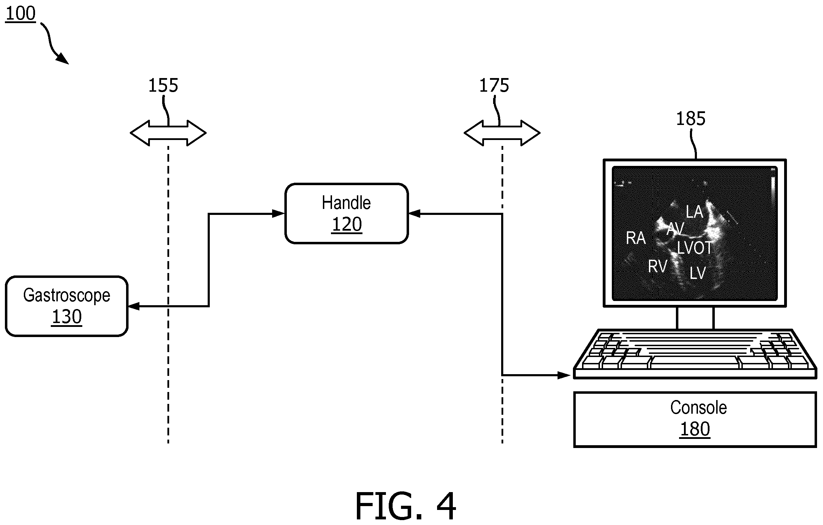

Fig. 4 depicts a diagrammatic view of an ultrasonic imaging system 100 according to an embodiment of the present disclosure. In some embodiments, the ultrasonic imaging system comprises a TEE imaging system. Referring to the embodiment of Fig. 4, the gastroscope 130 is in communication with the handle 120 via a probe interface 155, and the handle 120 is in communication with a console 180, via a console interface 175. The probe interface 155 may include a combination of a gastroscope-mating interface and a handle-mating interface, while the console interface 175 may include a console interface cable and a console interface connector, such as those described in relation to Fig. 3. The gastroscope 130 may obtain ultrasonic imaging data, transmit the ultrasonic imaging data to the handle 120 via the probe interface 155, and the handle 120 may transmit the ultrasonic imaging data to the console 180 via the console interface 175. Additionally, the gastroscope 130 may receive control data from the console 180 via the handle 120 to control various aspects of a TEE scan, such as one or more firing sequences of an ultrasonic transducer array, and the movement or flexion of the distal portion 139 of the gastroscope 130.

In some embodiments, the gastroscope 130 transmits the ultrasonic imaging data to the console 180 through the handle 120. In some embodiments, the console interface 175 may be situated at a proximal portion of the handle 120, such as the proximal end of the handle 120. In some embodiments, the console interface 175 may comprise at least one of a USB interface or a pogo pin interface. In other embodiments, the console interface 175 may comprise a cable permanently fixed to the handle 120. As will be illustrated in more detail below, in some embodiments, the handle 120 includes electronic components to modulate or process the ultrasonic imaging data being transmitted to the console 180.

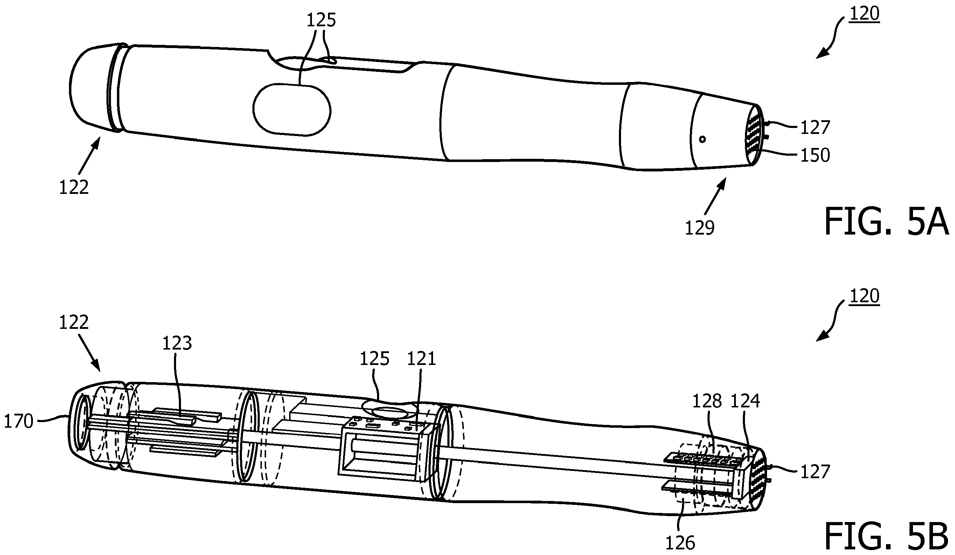

Figs. 5A and 5B depict a handle 120 of a TEE probe 110 according to an embodiment of the present disclosure. The handle of Figs. 5A and 5B may comprise similar or identical components as the handle depicted in Fig. 3, such as a proximal portion 122, a distal portion 129, and a handle-mating interface 150. The handle-mating interface 150 may comprise a pogo pin interface including an array of pogo pins, and a latch 127 configured to removably couple to a gastroscope, such as the gastroscope 130 of Fig. 3. The handle 120 may also comprise one or more user input selectors 125 configured to receive a user input and transmit a user input signal. In various embodiments, the handle 120 can include one, two, three, four, or more user input selectors 125. For example, the user input may comprise clicking a button. The button click may be transformed into a signal to initiate an ultrasound scanning protocol, or to control movement of the gastroscope 130. In other embodiments, the user input selectors 125 may include electronic buttons, dials, capacitive touch sensors, levers, switches, knobs, joysticks, etc.

Fig. 5B depicts a partially transparent view of the handle 120 of Fig. 5A. The handle 120 may comprise various electronics configured to control one or more aspects of a transesophageal echocardiogram. The handle 120 of Fig. 5B comprises a microcontroller 121 in communication with a console-mating interface 170 at the proximal portion 122 of the handle 120, the handle-mating interface 150 at the distal portion 129 of the handle 120, and the user input selectors 125. The microcontroller 121 can receive an electrical signal, such as a command signal, from a console via the console-mating interface 170, the user input selectors 125, or both. The microcontroller 121 may receive and transmit the electrical signal to the corresponding components of the TEE probe 110, such as the gastroscope 130. The handle 120 also comprises a power supply 123 configured to provide power to various components of the TEE probe 110. In the embodiment of Fig. 5B, the handle 120 comprises a beamformer 128, a scan controller 126, and a signal processor 124 at the distal portion 129 of the handle 120. The beamformer 128 may be configured to receive ultrasonic imaging data from an ultrasonic transducer, and process the ultrasonic imaging data to construct an ultrasonic image. The signal processor 124 may be configured to further process the ultrasonic imaging data to be displayed to a physician. The scan controller 126 may control various aspects of the ultrasonic imaging procedure carried out by an ultrasonic transducer, such as the frequency, pulse amplitude, and pulse transmission timing, echo reception timing, scan line pattern progression, etc.

Although the beamformer 128, signal processor 124, and scan controller 126 of the embodiment of Fig. 5B are disposed at the distal portion 129 of the handle 120, it is within the scope of this disclosure to position any of the beamformer 128, signal processor 124, or scan controller 126, in other areas of the of the TEE probe 110, such as the gastroscope 130, or the proximal portion of the handle 120. In some embodiments, one or more of these components may be part of an external console (e.g., console 180 of Fig. 4). In other embodiments, the handle 120 may be coupled to a console interface cable. In still other embodiments, the console interface cable may be permanently attached to the handle 120, or integrally formed with the handle 120. In some embodiments, the handle may comprise a battery in communication with the power supply 123 and configured to provide power to one or more electrical components of the TEE probe, such as the microcontroller 121, the scan controller 126, and the gastroscope 130.

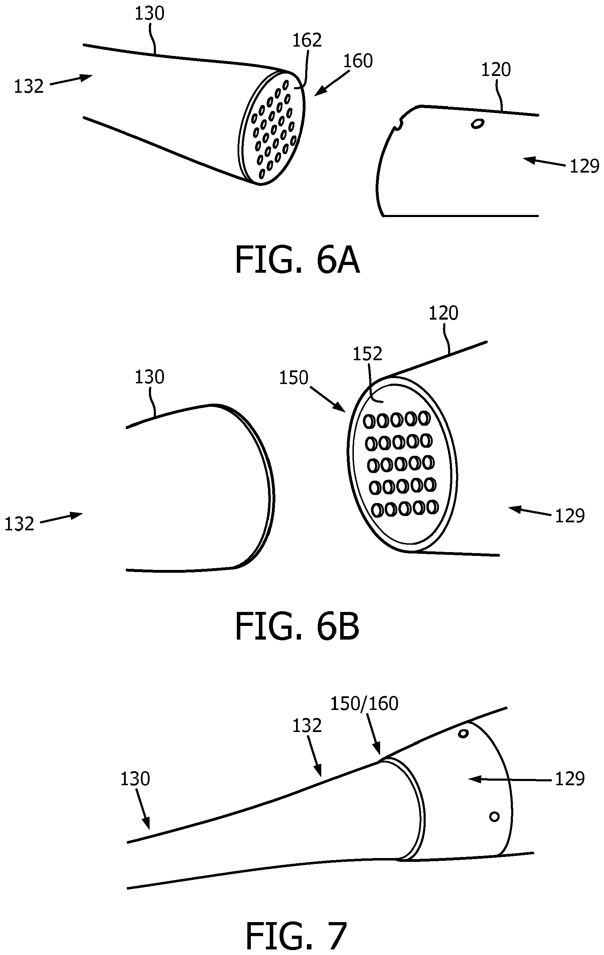

Figs. 6A and 6B depict perspective views of a gastroscope-mating interface 160 of a gastroscope 130 and a handle-mating interface 150 of a handle 120 of a TEE probe 110 according to one embodiment of the present disclosure. As shown in Fig. 6A, the gastroscope-mating interface 160 may be disposed at a proximal portion 132, such as a proximal end, of the gastroscope 130 and may comprise an array of female pogo connectors 162. The female pogo connectors 162 may correspond to an array of pogo pins 152 on the handle-mating interface 150, as shown in Fig. 6B. When coupled, the handle-mating interface 150 and the gastroscope-mating interface 160 may be configured to transmit electrical signals from the gastroscope 130 to electrical components of the handle 120, and vice versa.

In this disclosure, transmit may refer to an electrical component directing, routing, or permitting passage of an electrical signal to another component of the device. For example, an electrical wire may transmit an electrical signal even though the electrical wire does not contain electronics that can execute commands or selectively route an electrical signal to various electrical components.

Although the gastroscope-mating interface 160 and handle-mating interface 150 of the embodiment of Figs. 6A and 6B include pogo pin interfaces 162, 152, this disclosure contemplates any detachable interface that provides for data transfer between the gastroscope 130 and the handle 120 or console 180. For example, in some embodiments, the gastroscope-mating interface 160 and handle-mating interface 150 comprise a USB interface. In other embodiments, the gastroscope-mating interface 160 and handle-mating interface 150 comprise keyed electrical connectors, plug and socket connectors, or any other suitable interface.

Fig. 7 depicts the handle 120 and gastroscope 130 of Figs. 6A and 6B in a coupled state. In this configuration, the gastroscope-mating interface 160 is coupled to the handle-mating interface 150 to secure the gastroscope 130 to the handle 120, and to allow electrical signals to pass between the gastroscope 130 and the handle 120. Electrical signals passing between the handle 120 and the gastroscope 130 may include instructions to control movement of the gastroscope 130 within the body of a patient, ultrasonic imaging data, and other signals. The coupling of the handle 120 to the gastroscope 130 may include a physical connection by way of a latch, such as the latch 127 depicted in Fig. 5A, or any other suitable method of securing removable components, such as a magnetic connection, or by use of a conventional electrical connection, such as a USB interface, plug and socket connector, etc.

TEE probes having detachable or modular components can eliminate some of the equipment redundancy required by conventional TEE probes that comprise an integral unit. For example, a TEE probe 110 according to an embodiment of the present disclosure may allow a physician to attach one of a number of gastroscopes 130 to a handle 120 of the TEE probe 110. A physician may use a variety of gastroscopes 130 in her practice that correspond to the varying anatomy and physiology of her patients (e.g., gastroscopes of different diameters and/or lengths), as well as the different types of imaging she desires to perform. As the same handle can couple to a variety of gastroscopes 130, the physician need not have a separate complete TEE probe unit, including a handle and console interface cable, for each gastroscope. Additionally, TEE probes 110 having detachable components may have advantages in the cleaning of the devices. Components that require one type of cleaning process, such as a gastroscope 130, can be cleaned separately from other components that do not require the same cleaning process. For example, a handle 120, which can be damaged by the cleaning processes of the gastroscope 130, can be cleaned using a cleaning process more appropriate for its design and components.

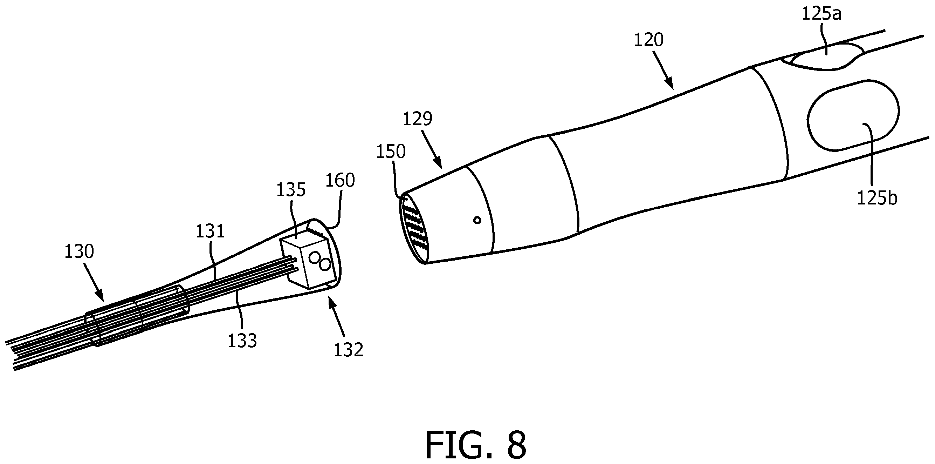

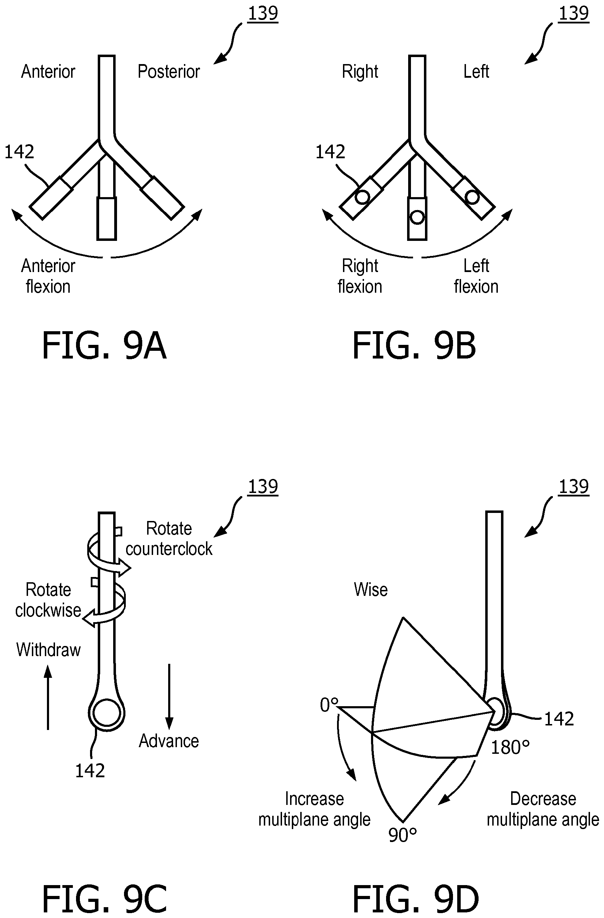

Fig. 8 depicts a partially transparent view of the TEE probe 110 of Fig. 3, with the gastroscope 130 separated from the handle 120. The gastroscope 130 includes a motor 135 coupled to, and configured to actuate, a first pull cable 131 and a second pull cable 133. The first pull cable 131 and second pull cable 133 may each comprise a plurality of individual cables or wires. The first and second pull cables 131, 133 may be coupled to different locations of the distal portion 139 of the gastroscope 130, such that when the motor 135 actuates or retracts the first and/or second pull cables 131, 133, the motor 135 can control various directions of movement of the distal portion 139 of the gastroscope 130. For example, as depicted in Fig. 9A, retracting and releasing the first pull cable 131 may function to control posterior and anterior flexion of the distal portion 139 of the gastroscope 130, while retracting and releasing the second pull cable 133 may function to control the right and left flexion of the distal portion 139 of the gastroscope 130.

Positioning the motor 135 within the gastroscope 130 may permit for a less complex interfacing or coupling between the gastroscope 130 and handle 120 while maintaining the operability, or maneuverability, of the gastroscope 130. Recalling Figs. 1 and 2, a conventional TEE probe 10 may position the movement controls 23, 25 for actuating the pull cables 31, 33 in the handle 20, which requires that the pull cables 31, 33 span from the distal portion of the gastroscope 30 to the movement controls 23, 25 in the handle 20. Such a configuration may not permit a modular configuration, such as the configuration of Fig. 8, in which the gastroscope 130 can be decoupled and recoupled to the handle 120 by the physician while preserving control of the distal portion 139 of the gastroscope 130 when the gastroscope 130 is coupled to the handle 120.

The gastroscope tip 140 includes a tip housing 141 that houses an ultrasonic transducer 142, and ultrasonic transducer wiring 143 in communication with the ultrasonic transducer 142 to carry ultrasonic imaging data to the handle 120. The gastroscope 130 also includes a first pull cable 131 and a second pull cable 133 that are coupled to, and span between, the handle 120 and the distal portion 139 of the gastroscope 130.

The first and second pull cables 31, 33 are coupled to a first movement control 23 and a second movement control 25, respectively. The first and second movement controls 23, 25 are coupled to the handle 20 and correspond to a direction of movement, or a degree of freedom, of the distal portion 39 of the gastroscope 30. For example, the first movement control 23 may correspond to a posterior and anterior flexion of the distal portion 39 of the gastroscope 30, while the second movement control 25 may correspond to a right and left flexion of the distal portion 39 of the gastroscope 30. Thus, by manipulating the first and second movement controls 23, 25, a user can control the movement and orientation of the distal portion 39 of the gastroscope 30 within the esophagus of a patient.

Although the gastroscope 130 shown in Fig. 8 comprises a motor 135 to control movement of the first and second pull cables 131, 133, the gastroscope 130 may comprise other mechanisms or actuators to control movement of the distal portion 139 of the gastroscope. Furthermore, although the gastroscope 130 of Fig. 8 comprises a first and second pull cable 131, 133, the gastroscope 130 may comprise additional pull cables, such as a third and a fourth pull cable. As described above, each pull cable may comprise a plurality of individual wires or cables configured to control movement of the distal portion 139 of the gastroscope 130.

Figs. 9A-9D depict distal portions 139 of gastroscopes 130 in various modes of movement and flexion. Fig. 9A shows a side view of a distal portion 139 of a gastroscope 130 in various modes of posterior and anterior flexion. Fig. 9B depicts a front-facing view of a distal portion 139 of a gastroscope 130 in various modes of right and left flexion. The anterior/posterior flexion and right/left flexion may be accomplished by retracting and releasing one or more pull cables corresponding to one or more modes of movement. As described above, the physician may control the anterior or posterior flexion of the distal portion 139 of the gastroscope 130 by retracting or releasing the first pull cable 131. As the first pull cable 131 may comprise a plurality of individual cables, controlling the anterior or posterior flexion of the distal portion of the gastroscope 130 may include retracting one of the individual wires of the first pull cable 131, while leaving another of the individual wires of the first pull cable 131 static.

Figs. 9C-9D depict additional modes of movement of the distal portion 139 of the gastroscope 130 and ultrasonic transducer 142. Fig. 9C depicts modes of physical movement, including withdrawal, advancement, and rotation, that can be achieved by manually adjusting the gastroscope 130, such as by rotating the handle to rotate the gastroscope 130 within the esophagus of the patient. A physician may also advance or withdraw the distal portion 139 of the gastroscope 130 within the esophagus of the patient by advancing or withdrawing the handle toward or away from the patient's esophagus. Fig. 9D depicts modes of electronic or beam angle movement related to emission of ultrasound energy by the transducer 142. The modes of movement depicted in Fig. 9D may not require physical manipulation of the distal portion of the gastroscope, such as the modes of movement depicted in Figs. 9A-9D. The electronic modes of movement of Fig. 9D may be accomplished by controlling one or more electronic aspects of an ultrasonic transducer array, such as the timing of firing and receive sequences.

Referring generally to Figs. 9A-9D, the illustrated various modes of movement and flexion of the distal portion 139 of the gastroscope 130 may be controlled by one or more user input selectors of the handle (e.g., 125a, 125b, Fig. 8). For example, in one embodiment, the anterior and posterior flexion of the distal portion 139 of the gastroscope 130 are controlled by a first user input selector 125a, while the left and right flexion are controlled by a second user input selector 125b. The rotating, advancing, and withdrawal of the distal portion 139 can be achieved by manually rotating, advancing, and withdrawing the handle 120 of the TEE probe 110. The electronic or beam angle movement may be manipulated or adjusted by a third user input selector. As described above, the user input selectors 125a, 125b may each comprise at least one of an electronic button, dials, capacitive touch sensors, levers, switches, knobs, joysticks, etc. In one embodiment, when a physician clicks an electronic button of the first user input selector 125a, the handle 120 receives the user input signal from the first user input selector 125a, and transmits a control signal to the gastroscope 130 via the handle-mating interface 150 and gastroscope-mating interface 160 to control the posterior and anterior flexion of the distal portion 139 of the gastroscope 130.

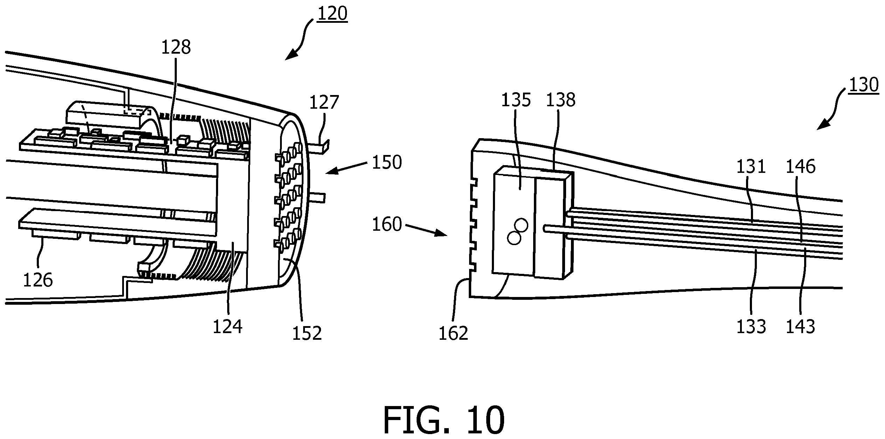

Fig. 10 depicts a cross-sectional view of the distal portion 129 of the handle 120 and the proximal portion 132 of the gastroscope 130 of the TEE probe 110 depicted in Fig. 3. The proximal portion 132 of the gastroscope 130 includes a force sensor controller 138 and a motor controller 134 in communication with the motor 135. The force sensor controller 138 may be in communication with a force sensor 145 (depicted in Fig. 11), and configured to receive a force sensor signal and perform an operation based on the force sensor signal.

For example, as a force is applied to the distal portion 139 of the gastroscope 130, the force sensor controller 138 may receive a force sensor signal including a detected force value from the force sensor 145, and compare the detected force value to a predetermined threshold. If the detected force value exceeds the predetermined threshold, the force sensor controller 138 can send instructions to the motor controller 134 to reduce or halt the motor's output, or to reverse the motor output to reverse the movement of the distal portion 139 of the gastroscope 130 to reduce or eliminate the force applied by the distal portion 139 of the gastroscope 130 to the patient's esophagus. Such a configuration may allow a physician to control movement of the distal portion 139 of the gastroscope 130 without applying force in excess of safety limits. Excessive force applied to the walls of the esophagus could result in injury to the patient.

The gastroscope of Fig. 10 further comprises ultrasonic transducer wiring 143 in communication with the ultrasonic transducer 142 and the gastroscope-mating interface 160. The ultrasonic transducer wiring 143 may carry ultrasonic imaging data from the ultrasonic transducer 142 to the gastroscope-mating interface 160 to be transmitted to the handle 120 and/or a console. The gastroscope 130 also comprises force sensor wiring 146 in communication with, and spanning between, the force sensor controller 138 and the force sensor 145. The force sensor wiring 146 may be configured to transmit force sensor data to the force sensor controller 138. In other embodiments, the force sensor wiring 146 may be in communication with the motor controller 134, wherein the motor controller 134 is configured to receive a force detection signal from the force sensor 145 to control the distal portion 139 of the gastroscope 130.

Fig. 11 depicts a cross-sectional view of the distal portion 139 of the gastroscope 130 of Fig. 3. In this embodiment, the distal portion 139 of the gastroscope 130 includes a gastroscope tip 140 at or near a distal end of the gastroscope 130. The gastroscope tip 140 comprises a tip housing 141 at an exterior of the gastroscope tip 140. The gastroscope tip 140 also includes an ultrasonic transducer 142, which may comprise an ultrasonic transducer array, and a force sensor 145 in communication with the tip housing 141 and configured to detect a force applied to the gastroscope tip 140 and/or to the distal portion 139 of the gastroscope 130. The force sensor 145 may comprise a flexible substrate positioned around the distal portion 139 of the gastroscope 130, or a flex circuit force sensor. The gastroscope tip 140 also comprises a microbeamformer 144 in communication with the ultrasonic transducer array of the ultrasonic transducer 142. The microbeamformer 144 may be configured to process or modify incoming electrical signals from the ultrasonic transducer array for use in creating an ultrasonic image. The microbeamformer 144 may also be used in combination with a corresponding beamformer (128, Fig. 5B) in the handle 120. In such a configuration, the microbeamformer 144 may eliminate some of the redundancy and electrical wiring required to transmit raw ultrasonic image data to from the ultrasonic transducer 142 to the console. Using a microbeamformer 144 in the gastroscope 130 may also help to reduce the complexity of the gastroscope-mating interface 160 and the handle-mating interface 150, such as by reducing the number of pogo pins 152, 162, required to transmit the ultrasonic imaging data.

Although some embodiments provide a microbeamformer 144 used in combination with a beamformer (128, Fig. 5B) in the handle 120, it is within the scope of this disclosure to provide a gastroscope 130 comprising a beamformer disposed within the gastroscope 130. Thus, all beamforming may take place within the gastroscope 130, or in other embodiments, beamforming may be shared between a microbeamformer of the gastroscope 130 and the beamformer of the handle 120. In some embodiments, all beamforming may take place within the handle 120. In other embodiments, the gastroscope 130 may not comprise a microbeamformer.

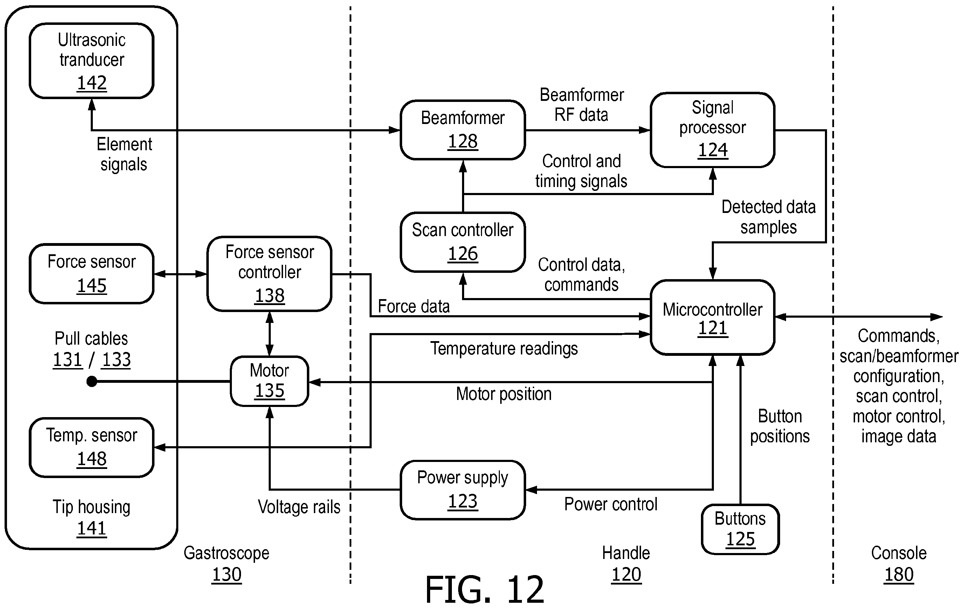

Fig. 12 depicts a schematic view of a TEE imaging system 100, according to one embodiment of the present disclosure. The TEE imaging system 100 of Fig. 12 may comprise similar or identical components as the embodiments shown in Figs. 3-11. As shown in Fig. 12, the TEE imaging system 100 may comprise a gastroscope 130 having a tip housing 141, a motor 135, pull cables 131, 133, an ultrasonic transducer 142, a force sensor 145, and a temperature sensor 148. These components may be arranged similarly to the configurations shown in Figs. 3-11, or may have a slightly different configuration.

As depicted in Fig. 12, a microcontroller 121 receives and transmits a variety of electrical signals to perform a variety of functions. The microcontroller 121 may receive commands from a console 180, or user input selectors, such as buttons 125, coupled to the handle 120. The microcontroller 121 may then process and relay those commands to the appropriate component, such as the power supply 123, the scan controller 126, and the motor 135. The microcontroller 121 may also receive signals from various components, such as the force sensor 145, the motor 135, and the signal processor 124. Signals received by the microcontroller 121 may include information such as force data, temperature data, or motor position data that may be processed by the microcontroller 121 as feedback to control and adjust various components of the TEE imaging system 100.

For example, the scan controller 126 may receive a command signal from the microcontroller 121 to begin a scan sequence, as well as control data and timing signals regarding the scan sequence to be performed. The scan controller 126 may transmit the control data and timing signals to the beamformer 128 and signal processor 124 to control various aspects of obtaining an ultrasound image. The beamformer 128 may transmit one or more firing signals to the ultrasonic transducer 142, causing the ultrasonic transducer 142 to emit ultrasonic energy in the form of ultrasonic waves into the patient's anatomy. The one or more firing signals may also comprise a command signal to measure or record echoes of the emitted ultrasonic waves. The ultrasonic transducer 142 may then measure the echoes, and translate the ultrasonic echoes into electrical signals comprising ultrasonic image data to be transmitted to the beamformer 128. The beamformer 128 may then process, modulate, or beamform the ultrasonic image data based on criteria from the scan controller 126, and transmit the beamformed ultrasonic image data to the signal processor 124 for further processing of the ultrasonic image data. The signal processor 124 may then transmit the processed ultrasonic image data to the microcontroller 121. On receiving processed ultrasonic image data from the signal processor 124, the microcontroller 121 may direct the processed ultrasonic image data to the console 180 via a console interface.

In another example, the microcontroller 121 sends a power control signal to the power supply 123 to apply a voltage or current to the motor 135 to control the function or output of the motor 135. The motor 135 may be coupled to one or more pull cables 131, 133, which may be coupled to the tip housing 141. As the motor 135 turns to actuate the pull cables 131, 133, the force sensor 145 may detect a force applied to a portion of the gastroscope, such as the tip housing 141, translate the detected force to an electrical signal, and transmit the electrical signal to the force sensor controller 138. The electrical signal from the force sensor 145 may include force data, such as an amount of force applied to the force sensor 145 and/or tip housing 141. The force sensor controller138 may perform a variety of functions, including comparing the force data to a threshold value, and transmitting at least one of a command signal and a detected force value to the microcontroller 121.

In some embodiments, when the force sensor controller 138 receives a detected force signal from the force sensor 145 indicating that a force applied to the force sensor 145 exceeds a threshold value, the force sensor controller 138 transmits a command signal to the microcontroller 121 to adjust a voltage or current applied to the motor 135 to adjust the output of the motor 135. In other embodiments, the microcontroller 121 receives the force data from the force sensor controller 138, compares the detected force value to a threshold value, and instructs the power supply 123 to adjust a voltage or current applied to the motor 135 to adjust the output of the motor 135. In still other embodiments, if the force sensor 145 detects a force that exceeds the predetermined threshold, the force sensor controller 138 may transmit an electrical signal directly to the motor 135 to adjust the output of the motor 135. In other embodiments, the microcontroller 121 may send instructions to the power supply 123 to halt power to the motor 135 to halt the motor's movement of the distal portion 139 of the gastroscope 130, or may instruct the motor 135 to reverse the movement of the motor 135 to return the distal portion 139 of the gastroscope 130 to a default position. The motor 135 may also send motor position data to the microcontroller 121 to allow a physician or user to predictably control movement of the distal portion 139 of the gastroscope 130.

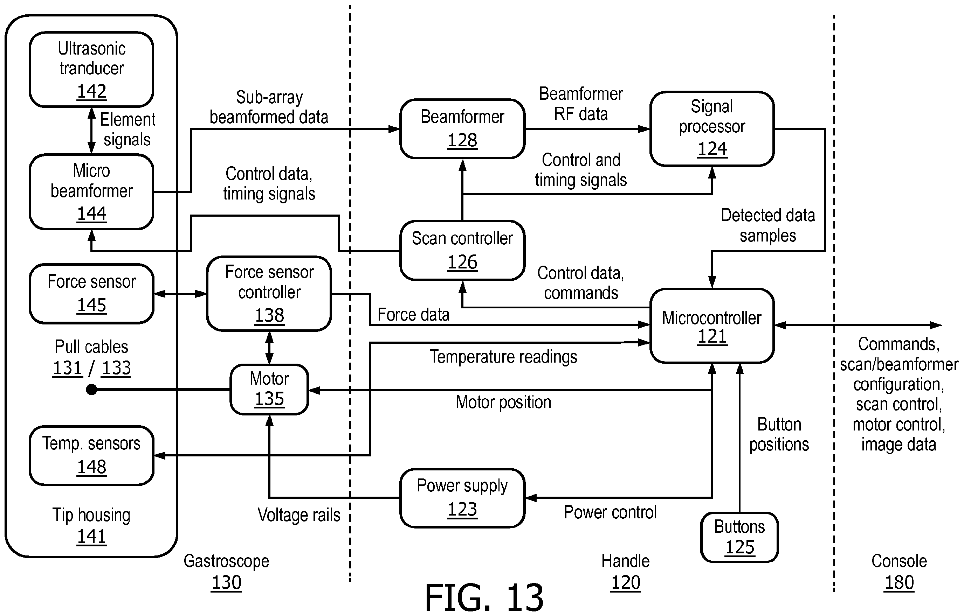

Fig. 13 depicts a schematic view of a TEE probe system, according to another embodiment of the present disclosure. Fig. 13 depicts similar components as the embodiment of Fig. 12, and further depicts a microbeamformer 144 positioned within the tip housing 141 and in communication with the scan controller 126, the beamformer 128, and the ultrasonic transducer 142. In other words, the function of the TEE probe of Fig. 13 may be similar to the TEE probe of Fig. 12 with the addition of the microbeamformer 144. The microbeamformer of Fig. 13 can be configured to process or modify raw ultrasonic image data obtained and transmitted by the ultrasonic transducer 142. As described above in connection with Fig. 11, the microbeamformer 144 may function to reduce the amount of data transmitted to the handle via the gastroscope-mating interface 160 and handle-mating interface 150. The microbeamformer 144 may also receive control data, such as timing signals, from the scan controller 126 to control the ultrasonic transducer 142. The beamformer 128 of the handle 120 may perform additional processing to the microbeamformed ultrasonic imaging data to prepare the data to be displayed for the physician.

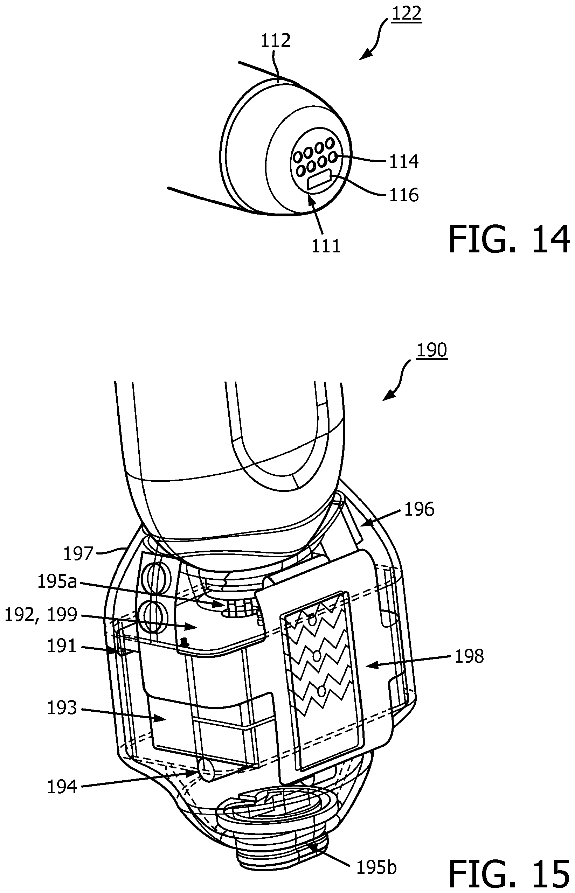

Turning to another aspect of the present disclosure, Fig. 14 depicts a proximal portion 122 of a handle 120 of a TEE probe 110 according to one embodiment of the present disclosure. The proximal portion 122 comprises a console interface 111 disposed at a proximal end of the handle 120 and configured to transmit data to a console. The console interface 110 includes a pogo pin interface 114 comprising an array of pogo connectors. The console interface also includes a key slot 117 configured to guide a mating connector into place. The pogo pin interface 114 may allow for removable coupling or attachment of the handle 120 to a console interface cable. Although the embodiment of Fig. 14 comprises a pogo pin interface 114, the handle 120 may comprise other interfaces or means of connecting to a console, such as a USB interface, a plug and socket interface, etc. A detachable USB cable, for example, can extend between the handle 120 and the console, and allow for a removable or separable connection of the cable to the handle 120, rather than a permanently attached cable as in some conventional TEE probe embodiments. As described below in connection to Fig. 15, the console interface may be configured to couple to a wireless module. In other embodiments, the console interface may be configured to couple to a charge cable to receive electrical power.

Fig. 15 depicts a wireless module 190 coupled to a handle of a TEE probe, according to one embodiment of the present disclosure. The wireless module 190 may comprise a housing 197 and a first module-mating interface 195a configured to couple to a console interface of the handle. In other embodiments, the wireless module 190 may be disposed within the handle, or may be permanently fixed to the handle 120.

In addition to the housing 197 and the first module-mating interface 195a, the wireless module of Fig. 15 comprises a radio module 191, a microcontroller 192, a battery 193, a power manager 199, a wireless communication element comprising an antenna 194, and a second module-mating interface 195b. The wireless module 190 may couple to the handle via the first module-mating interface 195a. In some embodiments, the first module-mating interface 195a comprises a pogo pin interface configured to couple to a pogo pin interface of the console interface of the handle. In other embodiments, the first module-mating interface 195a may comprise a USB interface, or any other suitable means for coupling to the handle.

The radio module 191 may be in communication with the antenna 194 and configured to receive a wireless signal from a console. In some embodiments, the wireless signal may comprise commands or instructions from the console to carry out one or more functions, such as a TEE scan. The radio module 191 may also be in communication with the microcontroller 192 to transmit the commands or instructions to various components of the handle 120 and/or gastroscope 130 via the console interface. For example, in some embodiments, the radio module 191 may receive instructions from a console 180 to control movement of the distal portion 139 of the gastroscope 130. The wireless module 190 may transmit the instructions to a controller in the handle 120. The controller may then instruct the power manager 199 to provide power (e.g., voltage, current) to a motor in the gastroscope or handle to actuate a pull cable configured to control movement of the distal portion of the gastroscope. In some embodiments, the microcontroller 192 may transmit the instructions directly to one or more components of the gastroscope, such as the motor or a motor controller. In some embodiments, the wireless module 190 may also comprise a user interface 198 and an audible indicator 196 configured to indicate a status or function of the wireless module 190. For example, in some embodiments, the audible indicator 196 may comprise a speaker, and may make an audible noise such as a beep to indicate that the wireless module 190 is correctly coupled to the handle.

Various components of the wireless module 190 may receive power from the battery 193. The battery 193 may be in communication with the power manager 199 configured to distribute power from the battery 193 to the other components of the wireless module 190. In some embodiments, the battery 193 of the wireless module 190 may also provide power to various electrical components of the handle and/or the gastroscope. For example, the motor of the gastroscope may receive power from the battery 193 of the wireless module 190. As in the embodiment shown in Fig. 15, the wireless module 190 may comprise a second module-mating interface 195b at a proximal portion of the wireless module 190. The second module-mating interface 195b may be configured to connect to a console interface cable or a charging cable. Thus, a TEE probe coupled to the wireless module 190 of Fig. 15 may be operated either wirelessly or by a wired connection to the console by a console interface cable.

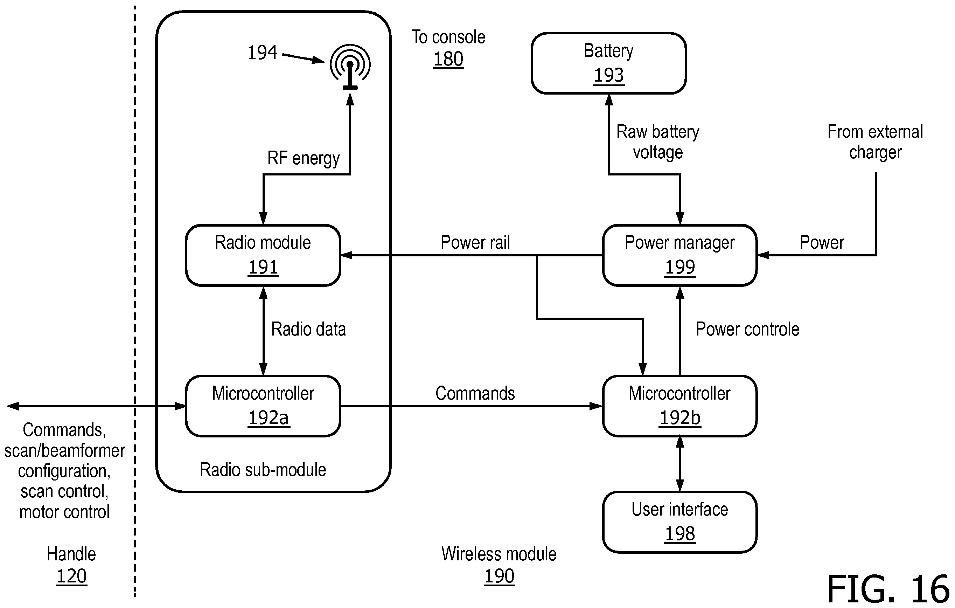

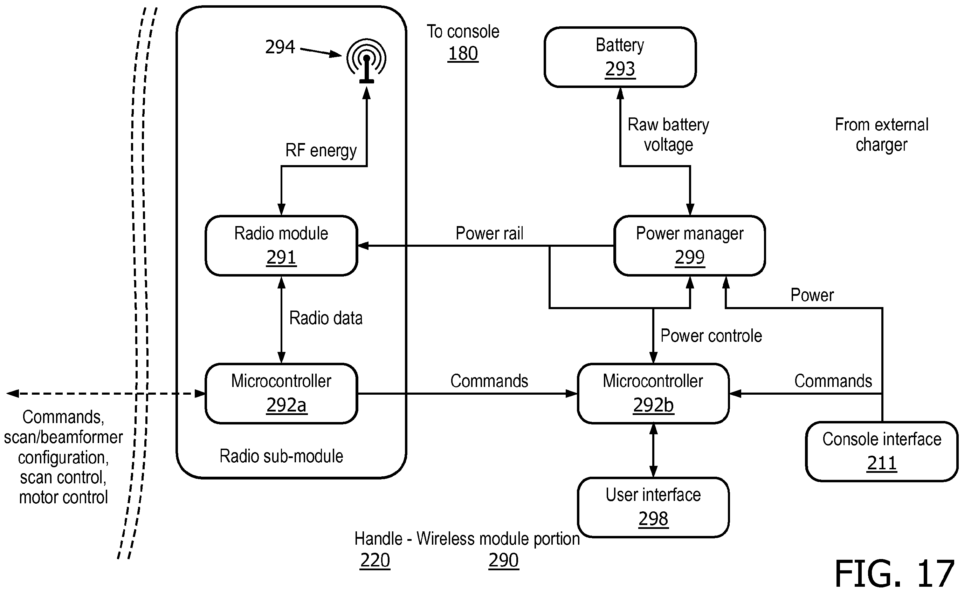

Figs. 16 and 17 are diagrammatic views of embodiments of a wireless module 190. The wireless module 190 of Fig. 16 may include similar or identical components as those depicted in Fig. 15, such as a radio module 191, a microcontroller 192a, and a battery 193. The battery 193 may be in communication with a power manager 199. The power manager 199 can be configured to distribute power to various components of the wireless module, such as the microcontroller 192b, and the radio module 191. In the embodiment of Fig. 16, the wireless module 190 may comprise a housing 197 that houses the electrical components of the wireless module 190. In other embodiments, such as the embodiment depicted in Fig. 17, a wireless module portion 290 may not comprise a housing, and may be disposed within a handle 220 of a TEE probe. In the embodiment of Fig. 17, the handle 220 may comprise a console interface 211 but not a first or second module-mating interface. The handle 220 may be configured to be controlled wirelessly by a console 180, or by a wired connection comprising a console interface cable coupled to the console interface 211 of the handle 220 (e.g., Fig. 14) and the console 180.

Referring to Fig. 16, the radio module 191 may receive, at the antenna 194, a radio signal from a console 180. The radio signal may comprise, for example, a command signal, and operating instructions. The radio module 191 may translate the radio signal to an electrical signal to be sent to the microcontroller 192a. On receipt of the electrical signal, the microcontroller 192a of the wireless module routes the electrical signal to the appropriate component, such as the handle 120. A battery 193 may supply electrical power to the radio module, microcontrollers 192a, 192b via a power manager 199. The power manager 199 may be configured to distribute an appropriate amount of electrical power to each of the components of the wireless module 190. Alternatively, an external charger may provide power to the various components of the wireless module 190 via the power manager 199. In some embodiments, the external charger may be configured to charge the battery 193 while simultaneously providing electrical power to the components of the wireless module 190. In other embodiments, the external charger only provides power to and charges the battery 193, and the battery 193 provides electrical power to the components of the wireless module. In still other embodiments, the battery 193 can provide power to components of other parts of a TEE probe system, such as components of the handle 120 and gastroscope 130. In some embodiments, the battery 193 of the wireless module 190 provides all electrical power necessary to operate the TEE probe system.

A user interface 198, which may comprise one or more user input selectors, such as buttons, dials, knobs, switches, levers, joysticks, touch sensors, etc., can be coupled to the housing of the wireless module 190 and configured to receive an input from a user. In some embodiments, the user may control one or more aspects of the wireless module 190 and/or TEE probe 110 by manipulating the user interface 198 (e.g., by pressing a button). The user interface 198 then sends a user input signal to the microcontroller 192b. The microcontroller 192b is configured to receive the user input signal and route the user input signal to the appropriate component, such as the power manager 199, or the microcontroller 192a. For example, the user interface 198 may be configured to receive an input command from a user to turn off one or more components of the wireless module 190. In other embodiments, the user interface 198 may be configured to control one or more aspects of a TEE scan, or the movement of the distal portion 139 of the gastroscope 130.

In one embodiment, microcontroller 192a of the wireless module 190 can receive ultrasound image data from handle 120. The microcontroller 192a can route the ultrasonic image data to the radio module 191, which then wirelessly transmits the ultrasonic image data to the console 180 via the antenna 194. Radio module 191 may be configured to translate the ultrasonic image data transmitted by the microcontroller 192a from an electrical signal into a radio signal.

Referring now to Fig. 17, a wireless module portion 290 of a handle 220 is shown that may include similar or identical components as the wireless module of Fig. 16. For example, wireless module portion 290 comprises a radio module 291 comprising an antenna 294, a microcontroller, a battery and power manager, a microcontroller, a console interface, and a user interface 298. In that regard, the wireless module portion 290 of the handle 220 may be configured to carry out similar or identical functions as described above with respect to the embodiment of Fig. 16. In the embodiment of Fig. 17, the wireless module portion 290 may be disposed within the handle 220, so as not to comprise a separable unit or attachment. In other words, the handle 220 comprises the wireless module portion 290 and the components therein. Thus, as described above, the wireless module portion 290 of the embodiment of Fig. 17 does not comprise a separate housing, or a first or second module interface.

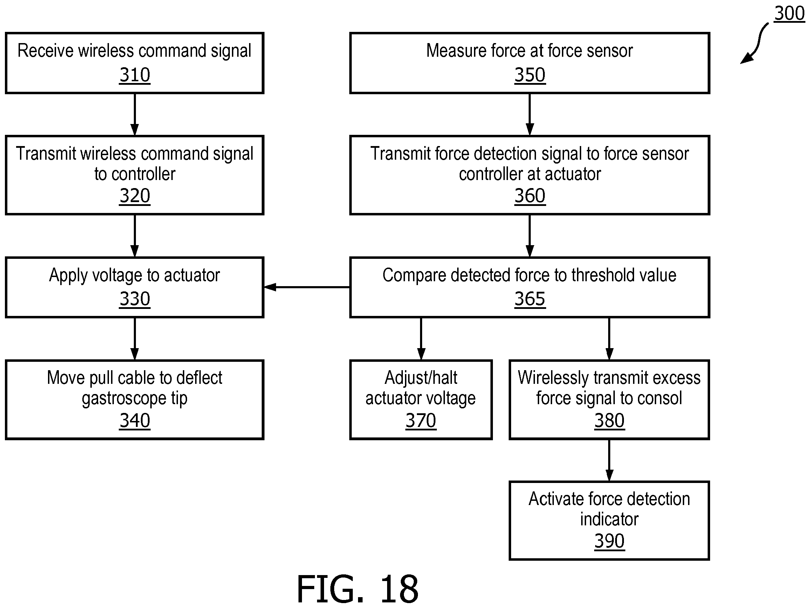

Fig. 18 is a flow diagram of one embodiment of a method 300 for wirelessly controlling a gastroscope of a TEE probe. In some embodiments, the steps of method 300 may be carried out by the TEE probe and associated components illustrated in Figs. 3-17. It is understood that the steps of method 300 may be performed in a different order than shown in Fig. 18, additional steps can be provided before, during, and after the steps, and/or some of the steps described can be replaced or eliminated in other embodiments.

In block 310, a TEE probe comprising a wireless module receives a wireless command signal from a console. The command signal may include instructions to move a gastroscope tip. The command signal may be wirelessly received by a radio module of the wireless module. In block 320, the radio module transmits the command signal to a controller. In block 330, the controller, on receiving the command signal, applies a voltage to an actuator, such as a motor, to activate the actuator. The application of voltage to the actuator by the controller may include instructing a power supply to distribute power to the actuator. In block 340, the actuator operates to retract a pull cable coupled to the gastroscope tip, or a distal portion of the gastroscope, to move or deflect the distal portion of the gastroscope.

As the gastroscope tip or distal portion of the gastroscope moves or deflects, the gastroscope tip may apply a force on the patient's esophagus, and vice versa. In block 350, a force sensor in the gastroscope tip measures a detected force applied to the gastroscope tip. In block 360, the force sensor transmits a force detection signal, which may include the amount of force applied to the gastroscope tip, to a force sensor controller in communication with the actuator and the wireless module. In block 365, the force sensor controller compares the detected amount of force to a predetermined threshold. In lock 370, if the detected force exceeds the threshold, the force sensor controller instructs the motor to adjust or halt the motor's output. In some embodiments, the step of instructing the motor to adjust or halt the motor's output may include instructing a power supply to decrease power distribute to the motor. In some embodiments, if the detected force exceeds the threshold, the force sensor controller may instruct the motor to reverse movement to return the gastroscope tip to a previous position within the patient's esophagus.

In block 380, when the force sensor controller determines that the detected force exceeds the predetermined force threshold, the forces sensor controller also transmits an excess force signal to the console. The transmission may be performed wirelessly by the wireless module. In block 390, when the console receives the excess force signal, the console activates a force detection indicator to indicate to a physician or user that the detected force exceeds the predetermined force threshold. Thus, the physician or user may be aware that too much force is being applied to the esophagus, and that the motor may halt movement of the gastroscope tip within the patient's esophagus. Such feedback may be helpful to a physician wirelessly controlling the TEE probe. Without such feedback, the physician may encounter difficulties in maneuvering the distal portion of the gastroscope within the patient's esophagus. In some embodiments, the force detection indicator may comprise a light, an icon on a screen, an alert sound, or a combination of indicators.

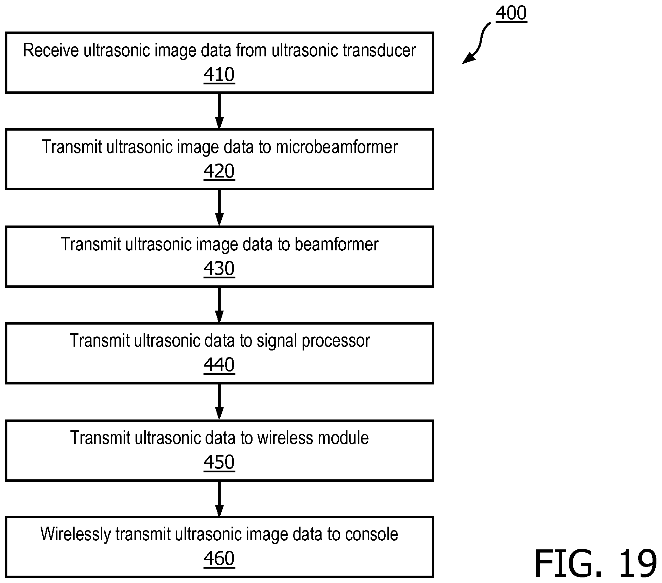

Fig. 19 is a flow diagram of one embodiment of a method for wirelessly transmitting ultrasonic image data to a console. In some embodiments, the steps of method 400 may be carried out by the TEE probe and associated components illustrated in Figs. 3-11. It is understood that the steps of method 400 may be performed in a different order than shown in Fig. 19, additional steps can be provided before, during, and after the steps, and/or some of the steps described can be replaced or eliminated in other embodiments.

In block 410, an ultrasonic transducer may obtain ultrasonic image data by emitting ultrasonic energy in the form of ultrasonic waves into the body of a patient. The ultrasonic transducer detects the reflected ultrasonic waves, or echoes, and transforms the reflected ultrasonic waves into electrical signals comprising the ultrasonic image data. In block 420, the ultrasonic transducer transmits the ultrasonic image data to a microbeamformer, which may be similar or identical to the microbeamformer 144 illustrated in Fig. 11. The microbeamformer receives processes, or microbeamforms, the ultrasonic image data, and in block 430, the microbeamformed ultrasonic imaging data is transmitted to a beamformer. In some embodiments, the microbeamformer is disposed within the gastroscope and the beamformer is disposed within the handle. In other embodiments, the beamformer may be located at the console. The beamformer receives the partially beamformed ultrasonic image data perform additional processing, or beamforming, on the ultrasonic imaging data to be displayed to a physician. In block 440, the beamformed ultrasonic imaging data is then received by a signal processor configured to further process the beamformed ultrasonic imaging data to create an ultrasonic image to be displayed to the physician. In some embodiments, the processing of the ultrasonic imaging data performed by the microbeamformer, the beamformer, and the signal processor, may reduce the amount of data that will be transmitted to the console. In block 450, the signal processor transmits the processed ultrasonic imaging data to the wireless module, and in block 460, the wireless module receives and wirelessly transmits the processed ultrasonic imaging data to the console.

Persons skilled in the art will also recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. A transesophageal echocardiography (TEE) probe, comprising:
a handle (120) comprising:
a proximal portion;
a distal portion (129); and
a handle-mating interface (150) disposed at the distal portion (129) of the handle (120); and
a gastroscope (130) coupled to the handle and configured to be positioned within an esophagus of a patient, the gastroscope comprising:
a proximal portion (132);
a distal portion (139);
an ultrasonic transducer disposed at the distal portion (139) of the gastroscope (130) and configured to obtain ultrasonic imaging data;
a motor (135) and a pull cable (133) coupled to the motor (135) and the distal portion (139) of the gastroscope, wherein the motor is configured to control movement of the distal portion (139) of the gastroscope; and
a gastroscope-mating interface (160) disposed at the proximal portion (132) of the gastroscope,
wherein the handle (120) and the gastroscope (130) are removably coupled via the handle-mating interface (150) and the gastroscope-mating interface (160), wherein, when the handle-mating interface and the gastroscope-mating interface are coupled, the handle-mating interface is configured to transmit an electrical signal to the gastroscope via the gastroscope-mating interface to control movement of the distal portion of the gastroscope.

2. The TEE probe of claim 1, wherein the ultrasonic transducer comprises an ultrasonic transducer array, and wherein the gastroscope (130) comprises a microbeamformer in communication with the ultrasonic transducer array.

3. The TEE probe of claim 2, wherein the microbeamformer is disposed at the distal portion (139) of the gastroscope.

4. The TEE probe of claim 1, wherein the ultrasonic transducer comprises an ultrasonic transducer array, and wherein the handle (120) comprises a beamformer in communication with the ultrasonic transducer array.

5. The TEE probe of claim 4, wherein the gastroscope comprises a microbeamformer in communication with the ultrasonic transducer array and the beamformer.

6. The TEE probe of claim 1, wherein the handle-mating interface and the gastroscope-mating interface comprise pogo pin interfaces.

7. The TEE probe of claim 6, wherein at least one of the handle or the gastroscope comprise a latch to secure the handle-mating interface and the gastroscope-mating interface.

8. The TEE probe of claim 1, wherein the handle comprises a controller in communication with the motor and the ultrasonic transducer.

9. The TEE probe of claim 1, wherein the gastroscope comprises a force sensor (145) disposed at the distal portion of the gastroscope and configured to detect a force applied to the distal portion of the gastroscope.

10. The TEE probe of claim 9, further comprising a force sensor controller (138) in communication with the force sensor (145) and the motor, the force sensor controller (138) configured to control actuation of the pull cable by the motor based on a force detected by the force sensor.

11. The TEE probe of claim 10, wherein the force sensor (145) comprises a flexible substrate positioned around the distal portion of the gastroscope.

12. The TEE probe of claim 1, wherein the handle comprises a console-mating interface at the proximal portion of the handle.

13. The TEE probe of claim 12, wherein the console-mating interface comprises at least one of a USB interface or a pogo pin interface.

## Patentansprüche

1. Transösophageale Echokardiographie (TEE)-Sonde, umfassend:
einen Griff (120), umfassend:
einen proximalen Abschnitt;
einen distalen Abschnitt (129); und
eine Griff-Paarungsschnittstelle (150), die am distalen Abschnitt (129) des Griffs (120) angeordnet ist; und
ein Gastroskop (130), das mit dem Griff gekoppelt ist und konfiguriert ist, um in einer Speiseröhre eines Patienten positioniert zu werden, wobei das Gastroskop umfasst:
einen proximalen Abschnitt (132);
einen distalen Abschnitt (139);
einen Ultraschallwandler, der an dem distalen Abschnitt (139) des Gastroskops (130) angeordnet ist und konfiguriert ist, um Ultraschallbilddaten zu erhalten;
einen Motor (135) und ein Zugkabel (133), die mit dem Motor (135) und dem distalen Abschnitt (139) des Gastroskops gekoppelt sind, wobei der Motor konfiguriert ist, um die Bewegung des distalen Abschnitts (139) des Gastroskops zu steuern; und
eine Gastroskop-Paarungsschnittstelle (160), die am proximalen Abschnitt (132) des Gastroskops angeordnet ist,
wobei der Griff (120) und das Gastroskop (130) lösbar über die Griff-Paarungsschnittstelle (150) und die Gastroskop-Paarungsschnittstelle (160) gekoppelt sind, wobei, wenn die Griff-Paarungsschnittstelle und die Gastroskop-Paarungsschnittstelle gekoppelt sind, ist die Griff-Paarungsschnittstelle konfiguriert, um ein elektrisches Signal über die Gastroskop-Paarungsschnittstelle an das Gastroskop zu übertragen, um die Bewegung des distalen Abschnitts des Gastroskops zu steuern.

2. TEE-Sonde nach Anspruch 1, wobei der Ultraschallwandler ein Ultraschallwandlerarray umfasst und wobei das Gastroskop (130) einen Mikrostrahlformer umfasst, der mit dem Ultraschallwandlerarray in Verbindung steht.

3. TEE-Sonde nach Anspruch 2, wobei der Mikrostrahlformer am distalen Abschnitt (139) des Gastroskops angeordnet ist.

4. TEE-Sonde nach Anspruch 1, wobei der Ultraschallwandler ein Ultraschallwandlerarray umfasst und wobei der Griff (120) einen Strahlformer umfasst, der mit dem Ultraschallwandlerarray in Verbindung steht.

5. TEE-Sonde nach Anspruch 4, wobei das Gastroskop einen Mikrostrahlformer umfasst, der mit dem Ultraschallwandlerarray und dem Strahlformer in Verbindung steht.

6. TEE-Sonde nach Anspruch 1, wobei die Griff-Paarungsschnittstelle und die Gastroskop-Paarungsschnittstelle Pogo-Stiften-Schnittstellen umfassen.

7. TEE-Sonde nach Anspruch 6, wobei mindestens einer von dem Griff oder dem Gastroskop eine Verriegelung umfasst, um die Griff-Paarungsschnittstelle und die Gastroskop-Paarungsschnittstelle zu sichern.

8. TEE-Sonde nach Anspruch 1, wobei der Griff eine Steuerung umfasst, die mit dem Motor und dem Ultraschallwandler in Verbindung steht.

9. TEE-Sonde nach Anspruch 1, wobei das Gastroskop einen Kraftsensor (145) umfasst, der an dem distalen Abschnitt des Gastroskops angeordnet ist und konfiguriert ist, um eine auf den distalen Abschnitt des Gastroskops ausgeübte Kraft zu detektieren.

10. TEE-Sonde nach Anspruch 9, ferner umfassend eine Kraftsensorsteuerung (138), die mit dem Kraftsensor (145) und dem Motor in Verbindung steht, wobei die Kraftsensorsteuerung (138) konfiguriert ist, um die Betätigung des Zugkabels durch den Motor basierend auf einer durch den Kraftsensor erfassten Kraft zu steuern.

11. TEE-Sonde nach Anspruch 10, wobei der Kraftsensor (145) ein flexibles Substrat umfasst, das um den distalen Abschnitt des Gastroskops herum positioniert ist.

12. TEE-Sonde nach Anspruch 1, wobei der Griff eine Konsole-Paarungsschnittstelle am proximalen Abschnitt des Griffs umfasst.

13. TEE-Sonde nach Anspruch 12, wobei die Konsole-Paarungsschnittstelle mindestens eine von einer USB-Schnittstelle oder einer Pogo-Stiften-Schnittstelle umfasst.

## Revendications

1. Sonde d'échocardiographie transœsophagienne (TEE), comprenant:
une poignée (120) comprenant:
une partie proximale;
une partie distale (129); et
une interface d'accouplement de poignée (150) disposée au niveau de la partie distale (129) de la poignée (120); et
un gastroscope (130) couplé à la poignée et configuré pour être positionné dans un œsophage d'un patient, le gastroscope comprenant:
une partie proximale (132);
une partie distale (139);
un transducteur ultrasonore disposé au niveau de la partie distale (139) du gastroscope (130) et configuré pour obtenir des données d'imagerie ultrasonore;
un moteur (135) et un câble de traction (133) couplé au moteur (135) et à la partie distale (139) du gastroscope, où le moteur est configuré pour commander le mouvement de la partie distale (139) du gastroscope; et
une interface d'accouplement du gastroscope (160) disposée au niveau de la partie proximale (132) du gastroscope,
où la poignée (120) et le gastroscope (130) sont couplés de manière amovible via l'interface d'accouplement de poignée (150) et l'interface d'accouplement de gastroscope (160), où, lorsque l'interface d'accouplement de poignée et l'interface d'accouplement de gastroscope sont couplées, l'interface d'accouplement de poignée est configurée pour transmettre un signal électrique au gastroscope via l'interface d'accouplement de gastroscope pour commander le mouvement de la partie distale du gastroscope.

2. Sonde TEE selon la revendication 1, dans laquelle le transducteur ultrasonore comprend un réseau de transducteurs ultrasonores, et dans laquelle le gastroscope (130) comprend un microformeur de faisceaux en communication avec le réseau de transducteurs ultrasonores.

3. Sonde TEE selon la revendication 2, dans laquelle le microformeur de faisceaux est disposé au niveau de la partie distale (139) du gastroscope.

4. Sonde TEE selon la revendication 1, dans laquelle le transducteur ultrasonore comprend un réseau de transducteurs ultrasonores, et dans laquelle la poignée (120) comprend un microformeur de faisceaux en communication avec le réseau de transducteurs ultrasonores.

5. Sonde TEE selon la revendication 4, dans laquelle le gastroscope comprend un microformeur de faisceaux en communication avec le réseau de transducteurs ultrasonores et le formeur de faisceaux.

6. Sonde TEE selon la revendication 1, dans laquelle l'interface d'accouplement de poignée et l'interface d'accouplement de gastroscope comprennent des interfaces de broche pogo.

7. Sonde TEE selon la revendication 6, dans laquelle au moins l'un entre la poignée ou le gastroscope comprend un verrou pour fixer l'interface d'accouplement de poignée et l'interface d'accouplement de gastroscope.

8. Sonde TEE selon la revendication 1, dans laquelle la poignée comprend un dispositif de commande en communication avec le moteur et le transducteur ultrasonore.

9. Sonde TEE selon la revendication 1, dans laquelle le gastroscope comprend un capteur de force (145) disposé au niveau de la partie distale du gastroscope et configuré pour détecter une force appliquée à la partie distale du gastroscope.

10. Sonde TEE selon la revendication 9, comprenant en outre un dispositif de commande de capteur de force (138) en communication avec le capteur de force (145) et le moteur, le dispositif de commande de capteur de force (138) étant configuré pour commander l'actionnement du câble de traction par le moteur sur la base d'une force détectée par le capteur de force.

11. Sonde TEE selon la revendication 10, dans laquelle le capteur de force (145) comprend un substrat flexible positionné autour de la partie distale du gastroscope.

12. Sonde TEE selon la revendication 1, dans laquelle la poignée comprend une interface d'accouplement de console au niveau de la partie proximale de la poignée.

13. Sonde TEE selon la revendication 12, dans laquelle l'interface d'accouplement de console comprend au moins l'une entre une interface USB ou une interface de broche pogo.
